(19) Europäisches Patentamt
European Patent Office
Office européen des brevets

(11) **EP 4 401 617 B1**

(12) **EUROPEAN PATENT SPECIFICATION**

(45) Date of publication and mention
of the grant of the patent:
**20.05.2026 Bulletin 2026/21**

(21) Application number: **22785991.5**

(22) Date of filing: **14.09.2022**

(51) International Patent Classification (IPC):
*A61B 5/00* *(2006.01)*    *A61N 1/36* *(2006.01)*
*A61N 1/05* *(2006.01)*    *A61N 1/372* *(2006.01)*

(52) Cooperative Patent Classification (CPC):
**A61N 1/372; A61B 5/6885; A61B 5/721;**
A61B 5/067; A61B 5/7405; A61B 5/7455;
A61B 46/10; A61B 2090/065; A61B 2505/05;
A61B 2562/00; A61B 2562/0219; A61N 1/0541;
A61N 1/36039

(86) International application number:
**PCT/EP2022/075526**

(87) International publication number:
**WO 2023/041581 (23.03.2023 Gazette 2023/12)**

(54) **MEDICAL HANDLING SYSTEM**

MEDIZINISCHES HANDHABUNGSSYSTEM

SYSTÈME DE MANIEMENT MÉDICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.09.2021 EP 21196884**

(43) Date of publication of application:
**24.07.2024 Bulletin 2024/30**

(73) Proprietor: **Medizinische Hochschule Hannover
30625 Hannover (DE)**

(72) Inventors:
• **RAU, Thomas Stephan
30853 Langenhagen (DE)**
• **BUDDE, Leon
30161 Hannover (DE)**
• **BÖTTCHER-REBMANN, Georg
30161 Hannover (DE)**
• **SCHELL, Viktor
30659 Hannover (DE)**

(74) Representative: **Kröncke, Rolf
Meissner Bolte
Patentanwälte Rechtsanwälte
Partnerschaft mbB
Plathnerstraße 3A
30175 Hannover (DE)**

(56) References cited:
**US-A1- 2006 161 138     US-A1- 2015 173 839
US-A1- 2015 223 897     US-A1- 2016 081 754**

**Description**

This disclosure relates to a medical handling system with a handling device for handling a medical device attached to the handling device into a body region of a patient, wherein the handling device has at least one force sensor for detecting a force transmitted from the medical device to the handling device in at least one measuring direction, the handling system having an evaluation unit for evaluating a force signal which is emitted by the force sensor and represents the force detected by the force sensor.

Medical handlings are performed on patients in various fields. The following is an example of the implantation of a cochlear implant, but the invention is also suitable for medical handlings in other areas.

The current standard surgical technique for inserting the electrode array of a cochlear implant is manual handling using forceps or tweezers. After exposure of the cochlea, the electrode array is manually positioned and advanced until it is either inserted sufficiently deep or strong, noticeable resistance causes the surgeon to abort the handling. Excessive forces during handling should be avoided because otherwise damage to the inner ear could occur.

There are already proposals to measure the insertion forces occurring during the insertion of a cochlear implant by means of a force sensor and to monitor them accordingly. Such an insertion system is known, for example, from WO 2009/124287 A1. In US 2015/0223897 A1 a sterile handle for controlling a robotic surgical system from a sterile field is disclosed. In US 2016/0081754 A1 an apparatus, systems and methods for precise guidance of surgical tools are disclosed.

It is an object of the invention to provide a medical handling system with force sensing of a force transmitted to a handling device, in which improved and more precise force measurement is possible, in particular of very small forces.

This object is achieved by a measuring device according to claim 1.

In another aspect described herein, a medical handling system of the type mentioned above is characterized in that the evaluation unit is set up to determine a compensated force signal which is corrected for gravity and/or acceleration influences acting on the force sensor at least in the at least one measurement direction, which influence the force signal. In this way, the gravity and/or acceleration influences impacting the force measurement by the force sensor can be compensated.

Studies have shown that in the case of medical handlings in sensitive areas of the body, e.g. in the inner ear or on an eye, even relatively small forces can cause damage to the patient, in particular forces of a magnitude that the user (surgeon) can hardly feel or differentiate manually. It is therefore necessary, for example, for the handling of cochlear implants to detect forces in the range below 100 mN (millinew-tons) with an accuracy of a few mN. For forces in this range, gravity effects and/or other acceleration effects acting on the force sensor can play a significant role. The proposed compensation of gravity and/or acceleration influences acting on the force sensor by means of the evaluation unit can significantly improve the measurement accuracy of the force signal. The invention as defined in the independent claim therefore opens up useful application possibilities in the field of medical handlings in sensitive areas of patients' bodies. The medical handling system is suited for intraoperative use during a medical treatment of a patient.

The force sensor is set up to detect a force transmitted from the medical device to the handling device in at least one measuring direction. The at least one measuring direction can be implemented such that the handling force can be accurately measured, e.g. a force in the longitudinal direction of the medical device. In case of the insertion of a cochlear implant, the insertion force transmitted from the medical device to the handling device (insertion device) can be measured.

The invention is suitable for manual use of the handling device by the user as well as for robotic handling. The invention is suitable for static use in a fixture. For example, the handling device may be an insertion device for an electrode array of a cochlear implant. In this case, the medical device may be an electrode array of a cochlear implant. The handling device may also be configured for other areas of a patient's body, such as for intubation, for handling of a catheter or an endoscope, for performing optical coherence tomography (OCT) in an eye, or for handling of any kind of medical device in a surgical procedure. The medical device attached to the handling device may be, for example, a surgical device, a tube, a probe, or the like. The handling device has a mechanical connection interface for attaching the medical device to the handling device. The handling device may be equipped with medical devices of different kinds through the mechanical connection interface.

For determining the compensated force signal, the evaluation unit can be supplied with a measured and/or calculated gravity and/or acceleration signal, e.g. via an interface, which represents the gravity and/or acceleration acting on the force sensor at least in the measurement direction and influencing the force signal. The gravity signal may originate from a sensor arranged on or in the handling device. The gravity and/or acceleration signal may originate from another device, e.g., a stereooptical navigation system used to track the handling device. If the handling device is guided by means of a robot, the gravity and/or acceleration signal can be determined, for example, from spatial orientation information of the robot arm. In general, for determining the compensated force signal the evaluation unit can therefore be supplied with measured and/or calculated spatial orientation information, e.g. via an interface, which indicates the spatial orientation of the handling device in space. From this, the evaluation unit can calculate such a gravity signal to determine the compensated force signal.

Depending on the way how the measured and/or calculated gravity and/or acceleration signal is generated, the evaluation unit may be supplied with additional data required for determining the compensated force signal, for example about the weight of the medical device and portions of the mechanical connection interface.

In an advantageous embodiment, the handling system is set up to detect a compensated force signal with a magnitude below 100 mN. A measuring accuracy of less than +/-10 mN can be achieved, or less than +/-5 mN, or less than +/-1 mN.

According to an advantageous embodiment of the invention, the handling device has at least one gravity sensor with which the gravity influences acting on the force sensor at least in the measuring direction can be determined in the form of a gravity signal, the evaluation unit being set up to determine the compensated force signal taking into account the gravity signal of the gravity sensor. This has the advantage that the gravity sensing is implemented directly in the handling device, whereby a high accuracy can be achieved. Such a gravity sensor can, for example, be designed as a commercially available acceleration sensor. This enables simple, precise and cost-effective determination of the gravity influences acting on the force sensor.

Therefore, it is possible that the handling device has at least one acceleration sensor with which the acceleration influences acting on the force sensor at least in the measuring direction can be determined in the form of an acceleration signal, the evaluation unit being set up to determine the compensated force signal taking into account the acceleration signal of the acceleration sensor

It is also possible to use a second force sensor for compensating the gravity and/or acceleration influences impacting the force measurement by the force sensor. In such case, the second force sensor can be connected to a weight simulation element which simulates the weight of the mechanical connection interface and portions of the medical device.

It is also possible that the handling device has more than one gravity sensor and/or acceleration sensor.

The determination of the compensated force signal taking into account the gravity and/or acceleration signal can, for example, be carried out mathematically as follows:

$$F_C = F_S - F_{ZP} + m_G(a - a_{ZP})$$

Here $F_C$ denotes the compensated force signal, $F_S$ the force signal of the force sensor, $F_{ZP}$ a zero-point force signal, which represents an offset in the measurement. $a$ is the gravity and/or acceleration signal of the gravity and/or acceleration sensor, $a_{ZP}$ is a zero-point signal of the gravity and/or acceleration sensor, i.e. an offset in the characteristic curve. The term $m_G$ represents the sum of the moving masses acting on the force sensor, which essentially means the total mass of the mechanical connection interface and portions of the medical device attached to the handling device.

According to an advantageous embodiment of the invention, the handling device has a multi-axis acceleration sensor by means of which the acceleration acting on the handling device in multiple spatial directions can be determined. This has the advantage that the spatial orientation of the handling device can be determined in several spatial axes at once, which in turn has the advantage that the current spatial orientation of the handling device can be displayed on a screen or other display device, for example, superimposed with an anatomical structure of the patient in the sense of an augmented reality display. For example, a multi-axis inertial measurement unit (IMU) can also be arranged on the handling device, in which a multi-axis rotation sensor (gyrometer) and/or a multi-axis magnetometer can also be arranged in addition to a multi-axis acceleration sensor. This makes it possible to determine the spatial position and spatial orientation of the handling device even more precisely.

According to an advantageous embodiment of the invention, the evaluation unit is set up to determine the compensated force signal by further considering gravity and/or acceleration signals and/or spatial orientation information occurring in a direction arranged at an angle to the measuring direction, e.g. with an angle greater than 5 degrees or an angle of about 90 degrees. This can further improve the accuracy of the determination of the compensated force signal. Depending on the design principle of the handling device and/or the force sensor, gravity and/or acceleration influences occurring at an angle to the measuring direction, for example, can falsify the signal of the force sensor. This influence can now also be compensated.

According to an advantageous embodiment of the invention, the handling system is arranged to display the compensated force signal on a display device, for example in real time. In this way, information about the forces transmitted to the patient in the area of a surgical intervention can be presented to the user simultaneously during the surgical intervention.

According to an advantageous embodiment of the invention, the handling system is arranged to output an acoustic, optical, and/or haptic feedback signal to the user depending on the compensated force signal. For example, there can be an acoustic, optical, and/or haptic feedback actuator on or in the handling device and/or any other part of the handling system. For example, one or more limit values can be stored in the handling system. If the compensated force signal reaches or exceeds one of these limit values, the feedback signal can be output to the user by activating such feedback actuator. If several limit values are available, different feedback signals can be output, for example, in a staggered manner for different force values, e.g. with increasing intensity for increasing forces.

It is possible, for example, to output a warning tone when a predefined limit value of the compensated force signal is exceeded, for example in the form of an acoustic reproduction of the compensated force signal, e.g. by increasing pitch, frequency modulation or the like. Also conceivable is a visual representation of the compensated force signal, e.g. by light sources, such as light emitting diodes, built into the handling device or another part of the handling system, which, for example, represent the intensity of the compensated force signal by changing colors. Haptic feedback is also conceivable, e.g. in the form of housing vibrations of the handling device or through a separate vibration device, e.g. on the user's arm. Likewise, visualization of the compensated force signal in the surgical microscope and/or visualization in the user's field of view is advantageously possible, e.g. as an augmented reality representation.

According to an advantageous embodiment of the invention, the handling system is arranged for storing the compensated force signal over time and/or for storing multi-axis spatial position and/or spatial orientation information of the handling device over time. This has the advantage that the compensated force signal and/or the multi-axis spatial position and/or spatial orientation information can be retrieved and checked for later verification of the medical handling process, for training purposes, and/or patient data documentation. For example, the stored data can be played back in the form of a video.

According to an advantageous embodiment of the invention, the handling device is an insertion device for inserting the medical device attached to the insertion device into a body region of a patient. In such case, the medical handling system can be a medical insertion system.

According to an advantageous embodiment of the invention, the insertion device has a mechanical connection interface for attaching the medical device in the form of an electrode array of a cochlear implant to the insertion device. This has the advantage that the insertion device can be used as an insertion tool for the electrode array of a cochlear implant.

For example, the invention is suitable for insertion force measurement in routine care of patients with cochlear implants. The invention is suitable for research into the relationship between measured forces during cochlear implant insertion and the patient's residual hearing retention. In addition, critical insertion forces and thresholds can be identified so that the cochlear implant insertion procedure can be improved in general.

The invention enables further advantages:

- Reliable and meaningful handling force measurement by compensation of motion artifacts by means of sensor data fusion with at least one gravity and force sensing system.

  - Can be used intraoperatively
  - Research tool to develop a better understanding of the relationship between handling forces and residual hearing retention.

- This enables realistic force feedback for users during handling for the

  - Control/monitoring of the handling process
  - early detection of mishandlings, damaging events
  - Adaptation of the handling process by reacting to the information provided, e.g. pause during insertion, slowing down of the movement, rotation of the electrode array, change of the handling angle.
  - Increase patient safety

- Enabling correlation between orientation change and handling force

  - Investigation of correlations between orientation change and handling force
  - If correlation is identified: Additional feedback about unfavorable / too strong movements to the user

In an independent aspect, the present invention relates to a measuring device according to claim 1 for measuring a mechanical physical quantity using a non-sterile sensor. The measuring device has a sterile barrier separating a non-sterile region from a sterile region. The non-sterile sensor is located in the non-sterile area of the measuring device. The measuring device has a transmission element for mechanically transmitting the mechanical physical quantity gathered in the sterile area to the non-sterile sensor. Thereby, the transmission element projects from the non-sterile sensor through an opening in the sterile barrier into the sterile area. The invention has the advantage that in a sterile environment, for example in a surgical or other medical environment, the measurement of mechanical physical quantities, for example of forces or force effects, can be carried out in a manner that is harmless to the patient. In this regard, the measuring device or at least parts of the measuring device, such as the sensor, need not themselves be sterilized, but may be sterilely enclosed by the sterile barrier, for example encapsulated therein. In this way, a general sterile concept is provided which can be used for sensory detection of mechanical quantities in sterile environments with little effort. The measuring device can be a medical handling system of the aforementioned kind. The transmission element can be a mechanical connection interface of the aforementioned kind.

The measuring device can be used, for example, to measure forces exerted by a surgical instrument on a patient's tissue. It is also possible to measure forces that occur when an implant is inserted inside the body as a result of contact with the surrounding tissue.

The measurement of the mechanical physical quantity, for example the force measurement, can advantageously be carried out through the opening in the sterile barrier. The mechanical physical quantity, for example the force, can thus be transmitted to the sensor unhindered and thus unmodified by other elements. In order that the opening in the sterile barrier does not lead to a violation of the sterility requirements in the sterile area, the sterile barrier formed in this way can be further enhanced by additional features, as explained hereinafter.

For example, the sterile barrier may comprise a flexible element, such as a sterile drape (e.g. foil, surgical drape), which envelops and shields at least the non-sterile parts of the measuring device from the sterile environment.

The sterile barrier is specially designed, particularly in the region of the opening, to minimize the risk of contamination of the sterile region by creating an angled path, for example in the sense of a labyrinth, through the opening in the sterile barrier. According to the invention, the angled path is in a gap between a sterile cover member, which covers the transmission element in the region of the opening, and the transmission element.

The risk of contamination to be avoided includes

I.) the escape of non-sterile particles/droplets from the non-sterile interior of the sterile barrier through the opening into the sterile area and thus may cause e.g. contamination of the sterile area, as well as
II.) the entry of droplets of blood, secretions, rinsing fluid or other media from the sterile area into the interior of the sterile barrier, which can contaminate the measuring device there and/or come into contact with non-sterile surfaces there and flow back into the sterile area.

The measurement of the mechanical physical quantity requires a mechanically fixed, and advantageously also detachable, connection between the detection location of the mechanical physical quantity (e.g. instrument tip, implant) and the measuring device.

In the case of a (sterile) instrument, this can be connected directly to the (non-sterile) measuring device, e.g. by screwing it on, clamping it, pressing it in or similar, and thus itself acts as a transmission element for the force measurement. In the case of a (sterile) implant, this can be held by a rigid implant holder, which is connected to the measuring device and thus serves as a transmission element for the acquisition of the mechanical measured variable.

The sterile encapsulation of the measuring device by the sterile barrier, e.g. in the form of a rigid, single or multi-part housing, a sterile drape, foil or the like, includes an opening to allow direct connection to the sensing location of the mechanical physical quantity. The invention is thus characterized by (at least) one opening in the sterile barrier.

The transmission element can be guided through the opening without contact to surrounding elements, as otherwise frictional forces between the sterile barrier and instrument or between the sterile barrier and the transmission element would superimpose and falsify the measurement. The opening enables the transfer of a movement through the sterile barrier to the non-sterile sensor, which is necessary for the measurement. The movement may be very small (e.g. a few $\mu$m), as in the case of a force sensor based on the principle of a deformation body and strain gauge. However, the movement can also be large, at least along the longitudinal axis of the transmission element, if the force measurement is combined with, for example, a linear positioning movement.

Also described herein is an advantageous assembly sequence of the components of the measuring device:

○ first the sterile barrier is attached to the measuring device
○ this results in a sterile outer surface and a space inside the sterile barrier which encloses non-sterile surfaces, such as outer surfaces of the measuring device as well as inner surfaces of the sterile barrier, in particular the non-sterile sensor
○ the sterile transmission element is passed through the opening in the sterile barrier, whereby the (non-sterile) inner surfaces within the sterile barrier that are still accessible through the opening are now in turn covered in a sterile manner
○ the geometric design of the transmission element, opening and sterile barrier already ensures at this stage that contact of non-sterile inner surfaces by the user is no longer possible

If the sterile encapsulation is not realized by a rigid housing alone, but by or with a flexible drape or foil, the gap geometry in the area of the opening must be realized by constructive design of the measuring device. This is possible, for example, by means of a projecting sleeve, opening socket or the like surrounding the mounting point for the transmission element, which is passed from the inside to the opening in the flexible sterile barrier or through the opening in the flexible sterile barrier and to which the foil or the drape can be temporarily fixed.

In the event that components of the measuring device, such as the above-mentioned projecting sleeve/opening socket are passed through, the non-sterile surfaces of the measuring device which are not yet sterilely covered by the sterile barrier must be covered by a further sterile part, e.g. in the form of a union nut, which thus forms part of the sterile barrier.

To minimize the risk of contamination, the gap in the remaining opening between the sterile barrier and the transmission element can be reduced by one or more of the following features:

∘ constructive minimization of the gap width to the smallest possible dimension

∘ longest possible gap length to create a high aspect ratio

∘ and in particular by increasing the gap length by "twisting" the gap path

Such twisted or angled gap path makes it possible that there is no direct straight line connection from the sterile outer area to the first non-sterile inner surface, so that a direct entry and impact of droplets/splashes is prevented, as such droplet flight cannot occur "around the corner". By changing the direction of the gap several times up to a meandering design, the protective effect of the gap guide can be maximized and the risk of contamination can be reduced towards zero.

In this regard, a portion of the gap formed entirely by sterile surfaces may reduce the contamination risk II described above. A part of the gap that is also formed by non-sterile surfaces can reduce the contamination risk I described above.

The sterile barrier may be supplemented by one or more of the following features:

∘ Splash guard in the form of a plate or collar-shaped component which can be mounted on the transmission element and covers the remaining gap between the transmission element and the sterile barrier, thus additionally preventing droplets from flying,

∘ Positive or negative pressure inside the sterile barrier to create a defined flow direction in the "gap labyrinth"; the flow direction can be inward or outward, depending on whether contamination risk I or contamination risk II is to be reduced.

The invention is explained in more detail below with reference to examples of embodiments using drawings.

Figure 1    shows a schematic diagram of an insertion system,

Figure 2    shows a sectional side view of a part of an insertion device.

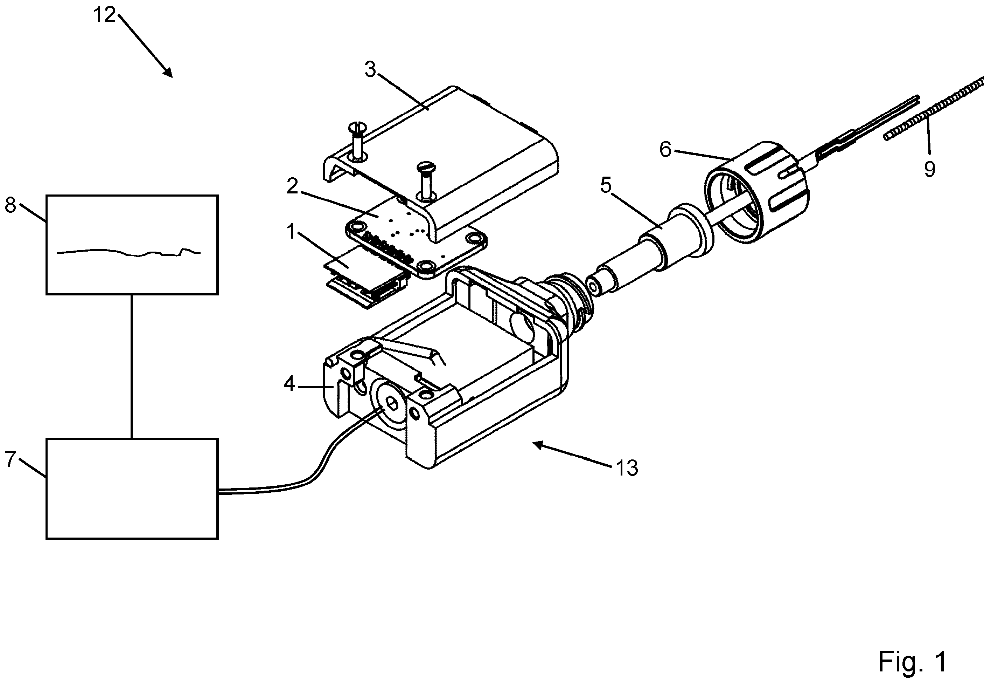

Figure 1 shows a medical insertion system 12 which has an insertion device 13, an evaluation unit 7, and a display device 8. The insertion device 13 can be designed, for example, as an insertion device to be held and guided manually. Alternatively, the insertion device 13 can also be guided by a robot.

The insertion device 13 has a multi-part housing with several parts, e.g. parts 3 and 4. The housing contains a force sensor 1 and a gravity sensor 2, e.g. in the form of a multi-axis multifunctional sensor unit in the sense of an inertial measurement unit (IMU). The force sensor 1 is mechanically coupled to a mechanical connection interface 5. A medical device 9 to be inserted into the patient by means of the insertion device 13 can be attached to the mechanical connection interface 5, for example, in the case of a cochlear implant, its electrode carrier. To achieve sterile conditions, part of the insertion device 13 and in particular the mechanical connection interface 5 can be covered by a closure cap 6.

The electrical or electronic components of the insertion device 13 are coupled to an evaluation unit 7 via electrical lines or wirelessly. The evaluation unit 7 is set up to evaluate the force signal emitted by the force sensor 1, the gravity signal emitted by the gravity sensor 2 and to determine a compensated force signal in which the gravity influences acting on the force sensor 1 are compensated by means of the gravity signal of the gravity sensor 2. The evaluation unit 7 is connected to a display device 8, for example a computer screen. The evaluation unit 7 is set up to display on the display device 8, for example, the compensated force signal in real time. The evaluation unit 7 can also output one or more feedback signals on the display device 8 or on other output components not shown here as a function of the compensated force signal, e.g. warning signals when certain predefined force values are reached.

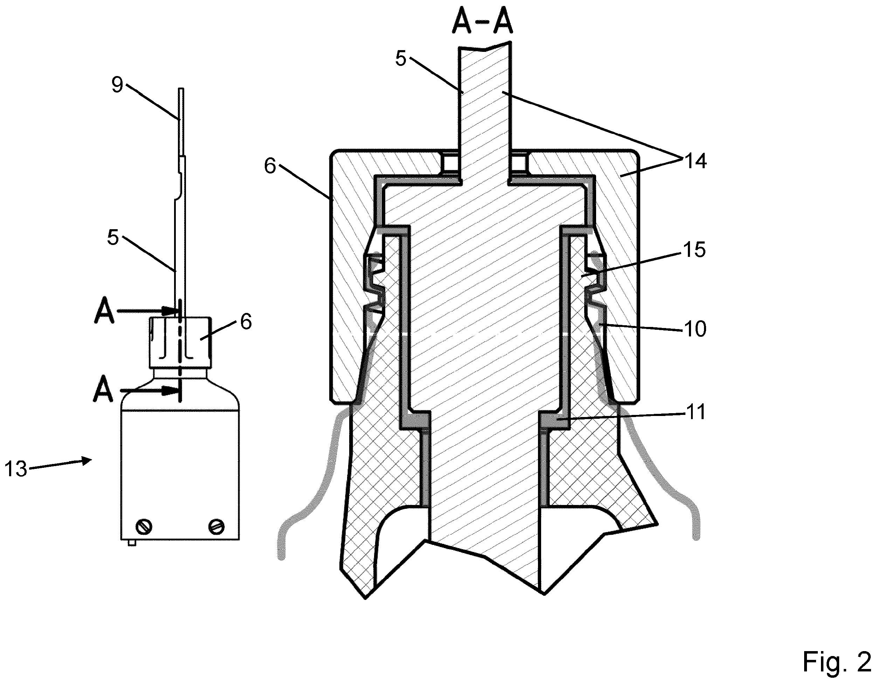

Figure 2 shows in the left hand area the insertion device 13 in a top view. In the right hand area of figure 2 the insertion device 13 facing the patient is shown in a sectional view along section line A - A. To enable the insertion device 13 to be used in accordance with regulations in the sterile operating room, some additional elements are added for separating sterile parts 14 from unsterile parts 15.

For example, some part of the insertion device 13 can be enclosed in a sterile cover (drape) 10. For example, a drape for use on endoscopes may be used. In the area of the mechanical connection interface 5, the sterile cover 10 is provided with a slight opening. To ensure a sterile barrier, the sterile closure cap 6 is screwed on in this area. The closure cap 6 thereby clamps the sterile cover 10 tightly and in this way prevents intraoperative slippage.

In addition, a labyrinth seal 11 may be located between the mechanical connection interface 5 and the housing 3, 4. Such a labyrinth seal 11 minimizes the risk of particles from falling out of the interior of the housing 3, 4 into the sterile area 14.

Since the insertion device 13 requires a through-opening from the sterile area where the medical device 9 is located to the force sensor within the housing 3, 4, it is required to provide a sterile concept which ensures that the sterile area of the medical device 9 is not contaminated through the opening from the area where the force sensor 1 is located.

The force sensor 1 has to be mechanically connected to the medical device 9, but shall not get in contact with other sterile elements. Therefore the non-sterile force sensor 1 needs to be separated from the sterile environment. This is done by the sterile cover 10 and the labyrinth seal 11. The insertion device 13 can be mounted in the following way.

1) Locating the insertion device 13 within the sterile cover 10;

2) Attaching the mechanical connection interface 5 on the insertion device 13;

3) Mounting the closure cap 6 on the insertion device 13;

4) Mounting the medical device 9 on the mechanical connection interface 5.

## Claims

1. Measuring device for a surgical or other medical environment for measuring a mechanical physical quantity using a non-sterile sensor (1), wherein the measuring device comprises a sterile cover member (10) providing a sterile barrier separating a non-sterile region (15) from a sterile region (14), wherein the non-sterile sensor (1) is located in the non-sterile region (15) of the measuring device, wherein the measuring device has a transmission element (5) for mechanically transmitting the mechanical physical quantity gathered in the sterile region (14) to the non-sterile sensor (1), whereby the transmission element (5) projects from the non-sterile sensor (1) through an opening in the sterile barrier into the sterile region (14), **characterized in that** the sterile barrier is achieved by an angled path through the opening, wherein the angled path is in a gap between the sterile cover member (10), which covers the transmission element (5) in the region of the opening, and the transmission element (5), wherein the transmission element (5) is guided through the opening without contact to surrounding elements.

2. Measuring device according to claim 1, wherein the sterile cover member (10) providing the sterile barrier comprises a flexible element, such as a sterile drape, which envelops and shields at least the non-sterile parts of the measuring device from the sterile environment.

3. Measuring device according to any one of the preceding claims, wherein the sterile encapsulation of the measuring device by the sterile cover member (10) providing the sterile barrier comprises a rigid, single or multi-part housing, a sterile drape or foil.

4. Measuring device according to any one of the preceding claims in the form of a medical handling system (12) with a handling device (13) for handling a medical device (9) attached to the handling device (13) within or relative to a body region of a patient, wherein the handling device (13) has, as the a non-sterile sensor, at least one force sensor (1) for detecting a force transmitted from the medical device (9) to the handling device (13) in at least one measuring direction, the medical handling system (12) having an evaluation unit (7) for evaluating a force signal which is emitted by the force sensor (1) and represents the force detected by the force sensor (1).

5. Measuring device according to claim 4, wherein the evaluation unit (7) is set up to determine a compensated force signal which is corrected for gravity and/or acceleration influences acting on the force sensor (1) at least in the at least one measurement direction, which influence the force signal.

6. Measuring device according to claim 5, wherein the handling device (13) has at least one gravity sensor (2) with which the gravity influences acting on the force sensor (1) at least in the measuring direction can be determined in the form of a gravity signal, the evaluation unit (7) being set up to determine the compensated force signal taking into account the gravity signal of the gravity sensor (2).

7. Measuring device according to claim 5 or 6, wherein the handling device (13) has at least one acceleration sensor (2) with which the acceleration influences acting on the force sensor (1) at least in the measuring direction can be determined in the form of a acceleration signal, the evaluation unit (7) being set up to determine the compensated force signal taking into account the acceleration signal of the acceleration sensor (2).

8. Measuring device according to any one of claims 4 to 7, wherein the handling device (13) is an insertion device for inserting the medical device (9) attached to the insertion device into a body region of a patient.

## Patentansprüche

1. Messvorrichtung für eine chirurgische oder andere medizinische Umgebung zum Messen einer mechanischen physikalischen Größe unter Verwendung eines nicht sterilen Sensors (1), wobei die Messvorrichtung ein steriles Abdeckelement (10) umfasst, das eine sterile Barriere bildet, die einen nicht sterilen Bereich (15) von einem sterilen Bereich (14) trennt, wobei der nicht sterile Sensor (1) in dem nicht sterilen Bereich (15) der Messvorrichtung angeordnet ist, wobei die Messvorrichtung ein Übertragungselement (5) aufweist, um die im sterilen Bereich (14) erfasste mechanische physikalische Größe mechanisch auf den nicht sterilen Sensor (1) zu übertragen, wobei das Übertragungselement (5) vom nicht sterilen Sensor (1) durch eine Öffnung in der sterilen Barriere in den sterilen Bereich (14) hineinragt, **dadurch gekennzeichnet, dass** die sterile Barriere durch einen abgewinkelten Weg durch die Öffnung erreicht wird, wobei sich der abgewinkelte Weg in einem Spalt zwischen dem sterilen Abdeckelement (10), das das Übertragungselement (5) im Bereich der Öffnung abdeckt, und dem Übertragungselement (5) befindet, wobei das Übertragungselement (5) ohne Kontakt zu umgebenden Elementen durch die Öffnung geführt wird.

2. Messvorrichtung nach Anspruch 1, wobei das sterile Abdeckelement (10), das eine sterile Barriere bildet, ein flexibles Element, wie beispielsweise ein steriles Tuch, umfasst, das zumindest die nicht sterilen Teile der Messvorrichtung umhüllt und vor der sterilen Umgebung abschirmt.

3. Messvorrichtung nach einem der vorstehenden Ansprüche, wobei die sterile Kapselung der Messvorrichtung durch das sterile Abdeckelement (10), das die sterile Barriere bildet, ein starres, ein- oder mehrteiliges Gehäuse, eine sterile Abdeckung oder Folie umfasst.

4. Messvorrichtung nach einem der vorstehenden Ansprüche in Form eines medizinischen Handhabungssystems (12) mit einer Handhabungsvorrichtung (13) zur Handhabung einer an der Handhabungsvorrichtung (13) befestigten medizinischen Vorrichtung (9) innerhalb oder relativ zu einem Körperbereich eines Patienten, wobei die Handhabungsvorrichtung (13) als nicht sterilen Sensor mindestens einen Kraftsensor (1) zum Erfassen einer von der medizinischen Vorrichtung (9) auf die Handhabungsvorrichtung (13) übertragenen Kraft in mindestens einer Messrichtung aufweist, wobei das medizinische Handhabungssystem (12) eine Auswerteeinheit (7) zum Auswerten eines vom Kraftsensor (1) abgegebenen Kraftsignals aufweist, das die vom Kraftsensor (1) erfasste Kraft repräsentiert.

5. Messvorrichtung nach Anspruch 4, wobei die Auswerteeinheit (7) dazu eingerichtet ist, ein kompensiertes Kraftsignal zu ermitteln, das zumindest in der mindestens einen Messrichtung um auf den Kraftsensor (1) einwirkende Schwerkraft- und/oder Beschleunigungseinflüsse korrigiert ist, die das Kraftsignal beeinflussen.

6. Messvorrichtung nach Anspruch 5, wobei die Handhabungsvorrichtung (13) mindestens einen Schwerkraftsensor (2) aufweist, mit dem die zumindest in der Messrichtung auf den Kraftsensor (1) einwirkenden Schwerkrafteinflüsse in Form eines Schwerkraftsignals ermittelbar sind, wobei die Auswerteeinheit (7) dazu eingerichtet ist, das kompensierte Kraftsignal unter Berücksichtigung des Schwerkraftsignals des Schwerkraftsensors (2) zu ermitteln.

7. Messvorrichtung nach Anspruch 5 oder 6, wobei die Handhabungsvorrichtung (13) mindestens einen Beschleunigungssensor (2) aufweist, mit dem die auf den Kraftsensor (1) zumindest in Messrichtung einwirkenden Beschleunigungseinflüsse in Form eines Beschleunigungssignals ermittelbar sind, wobei die Auswerteeinheit (7) dazu eingerichtet ist, unter Berücksichtigung des Beschleunigungssignals des Beschleunigungssensors (2) das kompensierte Kraftsignal zu ermitteln.

8. Messvorrichtung nach einem der Ansprüche 4 bis 7, wobei die Handhabungsvorrichtung (13) eine Einführvorrichtung zum Einführen der an der Einführvorrichtung befestigten medizinischen Vorrichtung (9) in einen Körperbereich eines Patienten ist.

**Revendications**

1. Dispositif de mesurage pour un environnement chirurgical ou autre environnement médical destiné à mesurer une quantité physique mécanique à l'aide d'un capteur non stérile (1), dans lequel le dispositif de mesurage comprend un élément de couverture stérile (10) fournissant une barrière stérile séparant une région non stérile (15) d'une région stérile (14), dans lequel le capteur non stérile (1) est situé dans la région non stérile (15) du dispositif de mesurage, dans lequel le dispositif de mesurage a un élément de transmission (5) destiné à transmettre mécaniquement la quantité physique mécanique, recueillie dans la région stérile (14), au capteur non stérile (1), grâce à quoi l'élément de transmission (5) se projette depuis le capteur non stérile (1) à travers une ouverture dans la barrière stérile jusque dans la région stérile (14), **caractérisé en ce que** la barrière stérile est terminée par un trajet en angle à travers l'ouverture, dans lequel le trajet en angle est un intervalle entre l'élément de couverture stérile (10), qui recouvre l'élément de transmission (5) dans la région de l'ouverture, et l'élément de transmission (5), dans lequel l'élément de transmission (5) est guidé à travers l'ouverture, sans contact avec des éléments environnants.

2. Dispositif de mesurage selon la revendication 1, dans lequel l'élément de couverture stérile (10) fournissant la barrière stérile comprend un élément flexible, tel qu'un drap stérile, qui enveloppe et protège au moins les parties non stériles du dispositif de mesurage, de l'environnement stérile.

3. Dispositif de mesurage selon l'une quelconque des revendications précédentes, dans lequel l'encapsulation stérile du dispositif de mesurage par l'élément de couverture stérile (10) fournissant la barrière stérile comprend un boîtier rigide unique ou en plusieurs parties, un drap ou une feuille stériles.

**4.** Dispositif de mesurage selon l'une quelconque des revendications précédentes sous la forme d'un système médical de manipulation (12) avec un dispositif de manipulation (13) destiné à manipuler un dispositif médical (9) attaché au dispositif de manipulation (13) à l'intérieur de ou relativement à une région corporelle d'un patient, dans lequel le dispositif de manipulation (13) a, en tant que capteur non stérile, au moins un capteur de force (1) destiné à détecter une forte transmise depuis le dispositif médical (9) au dispositif de manipulation (13) dans au moins une direction de mesurage, le système médical de manipulation (12) ayant une unité d'évaluation (7) destinée à évaluer un signal de force qui est émis par le capteur de force (1) et qui représente la force détectée par le capteur de force (1).

**5.** Dispositif de mesurage selon la revendication 4, dans lequel l'unité d'évaluation (7) est configurée pour déterminer un signal de force compensé qui est corrigé en cas d'influences de gravité et/ou d'accélération agissant sur le capteur de force (1) au moins dans ladite au moins une direction de mesurage, qui influencent le signal de force.

**6.** Dispositif de mesurage selon la revendication 5, dans lequel le dispositif de manipulation (13) a au moins un capteur de gravité (2) avec lequel les influences de gravité agissant sur le capteur de force (1) au moins dans la direction de mesurage peuvent être déterminées sous la forme d'un signal de gravité, l'unité d'évaluation (7) étant configurée pour déterminer le signal de force compensé en prenant en compte le signal de gravité du capteur de gravité (2).

**7.** Dispositif de mesurage selon la revendication 5 ou 6, dans lequel le dispositif de manipulation (13) a au moins un capteur d'accélération (2) avec lequel les influences d'accélération agissant sur le capteur de force (1) au moins dans la direction de mesurage peuvent être déterminées sous la forme d'un signal d'accélération, l'unité d'évaluation (7) étant configurée pour déterminer le signal de force compensé en prenant en compte le signal d'accélération du capteur d'accélération (2).

**8.** Dispositif de mesurage selon l'une quelconque des revendications 4 à 7, dans lequel le dispositif de manipulation (13) est un dispositif d'insertion destiné à insérer, dans une région corporelle d'un patient, le dispositif médical (9) attaché au dispositif d'insertion.

Fig. 1

Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009124287 A1
- US 20150223897 A1
- US 20160081754 A1